# EUROPÄISCHE PATENTANMELDUNG

(11) **EP 1 658 843 A1**
(43) Veröffentlichungstag der Anmeldung: **24.05.2006**
(21) Anmeldenummer: 05022107.6
(22) Anmeldetag: 11.10.2005
(51) Int. Cl.: A61K 31/045, A61P 3/06

(54) **Verwendung von langkettigen Alkoholen zur effektiven Senkung erhöhter Cholesterinspiegel**

(30) Priorität: 19.11.2004 DE 102004055858
(71) Anmelder: Clariant Produkte (Deutschland) GmbH, 65929 Frankfurt am Main (DE)
(72) Erfinder: Kammermeier, Bernhard, Dr., 80687 München (DE); Heinrichs, Franz-Leo, Dr., 86456 Gablingen (DE); Krendlinger, Ernst, Dr., 86316 Friedberg (DE); Danner, Barbara, 82278 Althegnenberg (DE)
(74) Vertreter: Paczkowski, Marcus

(57) **Zusammenfassung**

Die Erfindung betrifft die Verwendung von langkettigen Alkoholen zur effektiven Senkung erhöhter Cholesterinspiegel, dadurch gekennzeichnet, dass die langkettigen Alkohole aus Montanwachsalkoholen, Guerbetalkoholen und deren Bienenwachsanaloga erhalten werden.

## Beschreibung

Die Erfindung betrifft die Verwendung von langkettigen Alkoholen zur effektiven Senkung erhöhter Cholesterinspiegel

Der Cholesterinspiegel lässt sich etwa wie folgt definieren:
Das HDL-Cholesterin (High-Density-Lipoprotein-Cholesterin) wird auch als das "gute" Cholesterin bezeichnet, da es die Gefäße vor Fettablagerungen zu schützen scheint. Bei der Beurteilung des Cholesterinspiegels muss man zwischen dem "guten" und dem "schlechten" Cholesterin unterscheiden. Cholesterin liegt im Blut hauptsächlich in zwei verschiedenen Transportformen vor. Das "gute" HDL transportiert das Cholesterin von den Gefäßen zur Leber. Damit schützt es die Gefäße und kann Arteriosklerose vorbeugen. Eine hohe Konzentration von HDL im Blut wäre also wünschenswert.

Das "schlechte" LDL (Low-Density-Lipoprotein-Cholesterin transportiert dagegen das Cholesterin aus der Peripherie zu den Zellen. Bei einem Überangebot kann das LDL Cholesterin abgeben, das sich dann an den Gefäßwänden ablagert und zu Arteriosklerose führen kann. Das Verhältnis von LDL zu HDL im Blut sollte also möglichst gering sein. Idealerweise sollte ein LDL-Spiegel < 130 Milligramm pro Deziliter und ein HDL-Spiegel von > 45 Milligramm pro Deziliter angestrebt werden.

Es ist bekannt, dass aliphatische langkettige Alkohole aus dem Wachs von Saccharum Officinarum (Policosanol) bei einer Dosierung von 10 bis 20 mg/Tag den Gesamtcholesterinspiegel um 17 bis 21 % und den LDL-Spiegel um 21 bis 29 % senken können, wobei zusätzlich die HDL-Werte um 8 bis 15 % steigen (E. Ernst, MMW Nr. 23/2002, Seite 20).

Die Summenformel der vorgenannten Wachsalkohole lautet H₃C-(CH₂)ₙ-CH₂-OH, wobei die Kettenlänge n von 24 bis 32 Kohlenstoffatomen variiert. Tierexperimentelle Untersuchungen ergaben dabei, dass dieses Substanzgemisch neben den genannten Effekten noch weitere antiatherogene u.a. positive gesundheitliche Effekte aufweist, wobei keine adversen Effekte gefunden wurden. Vergleichsstudien zeigen eine zu herkömmlichen Statinen (verglichen wurden Simvastatin und Pravastatin) äquivalente Wirkung bei fehlendem Nebenwirkungsprofil im Dreijahresvergleich.

Nachteilig bei den aliphatischen langkettigen Alkoholen aus dem Wachs von Saccharum Officinarum sind jedoch natürliche Schwankungen der Produktqualität, der Alkoholzusammensetzung (Kettenlängenverteilung) und anderer qualitätsbestimmender Merkmale wie sie bei Substanzen aus erneuerbaren Rohstoffen naturbedingt auftreten.

Die vorgenannten Policosanole können zudem nur in sehr geringen Mengen (wenige Prozent) nach aufwendigen phys.-chemischen Gewinnungs- und Reinigungsmethoden aus dem entsprechenden natürlichen Rohstoff gewonnen werden.

Die Produkte sind nur sehr schwierig frei von Nebenprodukten herstellbar, wie sie in solchen komplexen Mischungen aus natürlichen Quellen stets auftauchen.

Insbesondere können aber auch mikrobielle Belastungen (Pilze und deren Mykotoxine, Bakterien, Protozoen und virale Komponenten) aus natürlichen Umweltbedingungen, oder auch Pestizide und Toxine anthropogenen Ursprungs, auftreten.

Bisher fehlt es an geeigneten langkettigen unverzweigten (und gegebenenfalls für physiologische Untersuchungen relevanten verzweigten) Alkoholen, die für die Senkung erhöhter Cholesterinspiegel zur Verfügung gestellt werden können.

Außerdem wäre es wünschenswert, dass die einzusetzenden Produkte keine mikrobiellen Belastungen aus natürlichen Umweltbedingungen und auch keine Pestizide oder Toxine anthropogenen Ursprungs aufweisen.

Gegenstand der Erfindung ist daher die Verwendung von langkettigen Alkoholen zur effektiven Senkung erhöhter Cholesterinspiegel, dadurch gekennzeichnet, dass die langkettigen Alkohole aus Montanwachsalkoholen, Guerbetalkoholen und deren Bienenwachsanaloga erhalten werden.

Bevorzugt werden die langkettigen Alkohole durch Verseifungsreaktionen aus den Wachsen erhalten.

Bevorzugt werden die langkettigen Alkohole auch durch oxidative Spaltung aus Montanwachs erhalten.

Bevorzugt weisen die langkettigen Alkohole keine mikrobiellen Belastungen auf.

Bevorzugt weisen die langkettigen Alkohole auch keine Belastungen durch Pestizide oder Toxine auf.

Vorteilhafterweise zeigen die aus den Montanwachsen abzuleitenden langkettigen unverzweigten Alkohole sowie die Guerbetalkohole sowie deren Bienenwachsanaloga im Vergleich zu den eingangs genannten Policosanolen einen analogen strukturellen Aufbau und deshalb ähnliche bis teilweise auch stärkere Wirkungen in Hinblick auf die Beeinflussung des Lipidmetabolismus.

Die genannten Montanwachsalkohole zeigen als langkettige unverzweigte Alkohole einen hochähnlichen gaschromatographischen Fingerprint wie die eingangs genannten Policosanole und wurden in gewebetoxikologischen Studien zusätzlich als untoxisch erkannt.

Der große Vorteil der Montanwachsalkohole und damit die technische Neuerung liegt zum einen in ihrer leichten und mengenmäßig praktisch "unbegrenzten" Verfügbarkeit und Zugänglichkeit aus Montanwachsen und der gewebetoxikologischen Unbedenklichkeit dieser Rohmaterialien und deren Derivate.

Weiterhin ist die Technik der qualitativ reproduzierbaren Gewinnung der genannten Wachse im Industriemaßstab gut etabliert und der Chemismus zur Freisetzung der Alkohole eine wohlbekannte und technisch sowie ökotoxikologisch ungefährliche Basisumsetzung.

Nach den bekannten industriellen Verfahren können die benötigten langkettigen Alkohole beispielsweise durch Verseifungsreaktionen vom Wachs gespalten und anschließend reinigendend extrahiert werden.

Das Montanwachs kann auch oxidativ über eine industriell zur Verfügung stehende Chromschwefelsäureoxidation gespalten werden und die resultierenden Montanwachssäuren reduktiv in die benötigten langkettigen Alkohole überführt werden.

Größere natürliche Schwankungen der Produktqualität, wie sie bei Substanzen aus erneuerbaren Rohstoffen - etwa bei den Policosanolen) naturbedingt auftreten, sind bei den aus diesen großindustriellen Verfahren erhältlichen genannten langkettigen Alkoholen nicht zu erwarten.

Guerbetalkohole sind gleichfalls aus gut zugänglichen Rohstoffen durch eine sehr einfache chemische Umsetzung zugänglich.

Diese langkettigen Alkohole zeigen mit ihrer zusätzlichen β-Verzweigung und besserer Steuerung/Einstellung der Gesamtkettenlänge eine weitere Möglichkeit der Beeinflussung des Lipidstoffwechsels im o.g. Sinne auf.

Da die metabolische Verwertbarkeit dieser Alkohole mit ihrer zusätzlichen β-Verzweigung aufgrund der Verzweigung schlechter als bei unverzweigten Analoga ist, resultiert ähnlich wie bei Montanwachsalkoholen eine geringe bis fehlende Möglichkeit der metabolischen Energiegewinnung ("fat replacers" in Literatur: "Natural and Synthetic Substances Related to Human Health", Pure and Appl. Chem. 2002, pages 1976 - 1977).

Sowohl Montanwachsalkohole als auch Guerbetalkohole können maßgeschneidert auf eine geforderte Kettenlängenverteilung synthetisiert werden (was bei den o.g. Policosanolen nicht der Fall ist) und sind frei von Nebenprodukten herstellbar, wie sie in komplexen Mischungen aus natürlichen Quellen stets auftauchen.

Insbesondere sind auch keine mikrobiellen Belastungen (Pilze und deren Mykotoxine, Bakterien, Protozoen und virale Komponenten) aus natürlichen Umweltbedingungen, oder auch Pestizide und Toxine anthropogenen Ursprungs zu erwarten.

Im Gegensatz zu den eingangs genannten Policosanolen liegt die Produktausbeute für Montanwachsalkohole aus Montanwachsen bei theor. 100 % (nach zusätzlicher Reduktion der Säurekomponente im Montanwachs), während die policosanole nach aufwendigen phys.-chemischen Gewinnungs- und Reinigungsmethoden bestenfalls im einstelligen Prozentbereich aus dem natürliche Rohstoff gewonnen werden.

Der Produktgehalt an Montanwachsalkoholen bzw. Guerbetalkoholen kann ohne die Notwendigkeit einer mikrobiologischen Produktkontrolle mittels Anwendung einer einfachen und gut etablierten Analytik bestimmt werden.

Weiterer Vorteil ist eine gesicherte Rohstoffbasis mit gleich bleibender Produktqualität und Ausbeutemenge ohne die natürlichen Schwankungen, denen ein Naturstoff üblicherweise unterliegt.

Ein weiterer Vorteil der vorliegenden Erfindung ist darin zu sehen, dass lediglich analoge (bis geringere) Dosismengen an Montanwachsalkoholen bzw. Guerbetalkoholen (oder auch ihrer Bienenwachsanaloga) im Vergleich zu Policosanolen aufgrund der sehr ähnlichen Molekülverteilungen und -strukturen zur Erzielung des Cholesterin senkenden Effekts unter Beibehaltung der oralen Applikationsform notwendig sind.

Auch treten beim Einsatz langkettiger Montanwachs- bzw. Guerbetalkohole keine kalorische Zusatzbelastungen aufgrund der fehlenden Metabolisierungsmöglichkeiten ("Natural and Synthetic Substances Related to Human Health", Pure and Appl. Chem. 2002, pages 1957 - 1985) auf.

## Patentansprüche

1. Verwendung von langkettigen Alkoholen zur effektiven Senkung erhöhter Cholesterinspiegel, **dadurch gekennzeichnet, dass** die langkettigen Alkohole aus Montanwachsalkoholen, Guerbetalkoholen und deren Bienenwachsanaloga erhalten werden.

2. Verwendung nach Anspruch 1, **dadurch gekennzeichnet, dass** die langkettigen Alkohole durch Verseifungsreaktionen aus den Wachsen erhalten werden.

3. Verwendung nach Anspruch 1, **dadurch gekennzeichnet, dass** die langkettigen Alkohole durch oxidative Spaltung aus Montanwachs erhalten werden.

4. Verwendung nach einem oder mehreren der Ansprüche 1 bis 3, **dadurch gekennzeichnet, dass** die langkettigen Alkohole keine mikrobiellen Belastungen aufweisen.

5. Verwendung nach einem oder mehreren der Ansprüche 1 bis 3, **dadurch gekennzeichnet, dass** die langkettigen Alkohole keine Belastungen durch Pestizide oder Toxine aufweisen.
